# EUROPEAN PATENT APPLICATION

(11) **EP 2 808 011 A1**
(43) Date of publication of application: **03.12.2014**
(21) Application number: 13169801.1
(22) Date of filing: 29.05.2013
(51) Int. Cl.: A61K 9/14, A61K 9/20, A61K 31/5377

(54) **Process for the preparation of a pharmaceutical composition comprising Rivaroxaban**

(71) Applicant: SANDOZ AG, 4056 Basel (CH)
(72) Inventor: Schwarz, Franz X., 6250 Kundl (AT); Kasper, Stephanie, 6250 Kundl (AT)
(74) Representative: Wichmann, Hendrik

(57) **Abstract**

The present invention relates to a process for the reparation of a pharmaceutical composition comprising rivaroxaban, wherein the process comprises a step of milling a mixture comprising rivaroxaban and at least one hydrophilic binder.

## Description

The present invention relates to a process for the preparation of a pharmaceutical composition comprising rivaroxaban, in particular to a simpler and more economic process for the preparation of a pharmaceutical composition comprising rivaroxaban compared to the processes of the prior art.

### Background prior art

Rivaroxaban (CAS Registry Number 366789-02-8; IUPAC Name: (S)-5-chloro-N-{[2-oxo-3-[4-(3-oxomorpholin-4-yl)phenyl]oxazolidin-5-yl]methyl} thiophene-2-carboxamide) is an oral anticoagulant. In particular, it is an orally active direct factor Xa inhibitor and is used for the prophylaxis of deep vein thrombosis, which may lead to pulmonary embolism, for stroke prophylaxis in patients with non-valvular atrial fibrillation, for the treatment of patients with deep vein thrombosis and pulmonary embolism and for long-term treatment to prevent recurrence. It has a relatively poor water solubility, which results in a low saturation solubility and a low dissolution rate. Therefore, rivaroxaban has to be processed before it is applied.

Lerk *et al.* (Lerk, Lagash, Fell, Nauta, Journal of Pharmaceutical Sciences, Vol 67, no. 7, July 1978, 935-939: "Effect of Hydrophilization of hydrophobicity Drugs on release rate from Capsules"; Lerk, Lagash, Lie-A-Huen, Broersma, Zuurman, Journal ofPharmaceutical Sciences, Vol 68, no. 5, May 1979, 634-638: "In Vitro and In Vivo Availability of Hydrophilized from phenytoin capsules") discloses a method for hydrophilization of hydrophobic drugs including mixing the hydrophobic drug with a methyl cellulose or hydroxyethylcellulose solution and subsequent drying. The resulting active ingredient is subsequently filled in hard gelatin capsules.

WO2005/060940 discloses a process for preparing a solid, orally administrable pharmaceutical composition comprising rivaroxaban in hydrophilized form, wherein first granulate containing rivaroxaban in hydrophilized form is prepared by wet granulation and the granulate is then, optionally with the addition of pharmaceutically suitable additives, converted into the pharmaceutical composition. Rivaroxaban is marketed as Xarelto^{®} in several countries.

However, there is still a need for simpler and more economic processes for the preparation of a pharmaceutical composition comprising rivaroxaban.

Therefore, it was an object of the present invention to provide a new process for the preparation of a pharmaceutical composition comprising rivaroxaban.

It was another object of the present invention to provide a process for the preparation of a pharmaceutical composition comprising rivaroxaban, wherein the obtained composition shows a satisfactory solubility and a satisfactory dissolution rate in water.

### Summary of the Invention

The present invention relates to a process for the preparation of a pharmaceutical composition comprising (S)-5-chloro-N-{[2-oxo-3-[4-(3-oxomorpholin-4-yl)phenyl]oxazolidin-5-yl]methyl}thiophene-2-carboxamide, wherein the process comprises a step of a) milling a mixture comprising (S)-5-chloro-N-{[2-oxo-3-[4-(3-oxomorpholin-4-yl)phenyl]oxazolidin-5-yl]methyll thiophene-2-carboxamide and at least one hydrophilic binder.

### List of figures

Figure 1: shows a light microscope image of untreated rivaroxaban (left) and a composition obtained according to a process as defined herein after the milling step (right).
Figure 2 and 4: show a comparison of different solubilities.
Figure 3 and 5: show a comparison of different dissolution rates.

### Definitions

The term "milling", also known as grinding, as used herein shall be understood as known in the art. In particular, it shall be understood as an operation in which material is crushed, pulverized, or reduced to powder by friction, especially by rubbing between two hard surfaces.

A "hydrophilic material" as used herein may refer to a material having a tendency to be solvated by water. Moreover, it may refer to a material that is attracted to, and tends to be dissolved by water and/or it may refer to a material that has an affinity for water; readily absorbing or dissolving in water. In particular, a "hydrophilic material" may have increased wettability properties. In addition, a "hydrophilic material" may have an HLB value of above 10.

A material is said to be "water soluble" if more than 0.1 g of that material dissolves in 100 mL water, preferably if more than 1 g of that material dissolves in 100 mL water. A "binder" as used herein refers to a material which holds the ingredients in a tablet together. Binders ensure that tablets and granules can be formed with required mechanical strength, and give volume to low active dose tablets.

A "hydrophilic binder" as used herein may refer to a material which after inclusion in a formulation increases the rate at which the particle separate, enhancing the available surface area so that wetting and dissolution can occur more rapidly, shortening the time needed for some poorly soluble drugs to go into solution.

An "excipient" as used herein may refer to a pharmacologically inactive substance formulated with the active ingredient of a medication. It may e.g. be a filler, a disintegrant, a lubricant, a binder, a colorant, a flavoring agent, a preservatives, or any mixture thereof.

The term "comprising" as used herein shall in every embodiment, aspect, example, or item be interpreted as encompassing all the specifically mentioned features, components or steps as well as optional, additional, unspecified ones. In addition, the term "comprising" as used herein shall also be interpreted to mean "consisting of", therewith defining for every embodiment, aspect, example, or item that no further features, components or steps are present.

### Detailed Description of the Invention

The present invention relates to a process for the preparation of a pharmaceutical composition comprising (S)-5-chloro-N-{[2-oxo-3-[4-(3-oxomorpholin-4-yl)phenyl]oxazolidin-5-yl]methyl} thiophene-2-carboxamide, wherein the process comprises a step of a) milling a mixture comprising (S)-5-chloro-N-{[2-oxo-3-[4-(3-oxomorpholin-4-yl)phenyl]oxazolidin-5-yl]methyl} thiophene-2-carboxamide and at least one hydrophilic binder.

After step a), the mixture may then be converted into the pharmaceutical composition, if appropriate with the addition of pharmaceutically suitable excipients. Particularly, the mixture may be converted into an orally administrable pharmaceutical composition.

Moreover, the process as defined herein does not include a step of granulating (S)-5-chloro-N-{[2-oxo-3-[4-(3-oxomorpholin-4-yl)phenyl]oxazolidin-5-yl]methyl} thiophene-2-carboxamide or granulating a mixture comprising (S)-5-chloro-N-{[2-oxo-3-[4-(3-oxomorpholin-4-yl)phenyl]oxazolidin-5-yl]methyl} thiophene-2-carboxamide.

The compositions obtained after a process as defined herein show satisfactory solubilities and a satisfactory dissolution rates in water compared to the compositions known in the prior art. Therefore, the process as defined herein allows for an economic preparation of a pharmaceutical composition comprising (S)-5-chloro-N-{[2-oxo-3-[4-(3-oxomorpholin-4-yl)-phenyl]oxazolidin-5-yl]-methyl} thiophene-2-carboxamide, wherein (S)-5-chloro-N-{[2-oxo-3-[4-(3-oxomorpholin-4-yl)phenyi]oxazolidin-5-yl]methyl} thiophene-2-carboxamide is simply milled together with a hydrophilic binder instead of being granulated.

### Step a)

The hydrophilic binder may be selected from the group consisting of hydroxypropyl methyl cellulose, cellulose methyl ether, hydroxypropyl cellulose, ethyl cellulose, carboxymethyl cellulose, galactomannan gum, xanthan, glycerides, acrylic and methacrylic copolymers with trimethylammoniomethyl acrylate, copolymers of dimethylaminomethacrylic acid and neutral methacrylic acid esters, polymers of methacrylic acid or methacrylic acid, and any mixtures thereof, wherein binders based on cellulose are preferred. In a preferred embodiment the binder is hydroxypropyl methyl cellulose. The binder may be water soluble. Typically, the binder is used in an amount of 2 to 10 wt.-%, preferably 3 to 8 wt.-%, more preferably 3 to 4 wt.-%, based on the total amount of the pharmaceutical composition. Particularly, the binder may be a cellulose, particularly hydroxypropyl methyl cellulose, and may be used in an amount of 3 to 4 wt.-%, based on the total amount of the pharmaceutical composition.

In the process as defined herein, (S)-5-chloro-N-{[2-oxo-3-[4-(3-oxomorpholin-4-yl)phenyl]oxazolidin-5-yl]methyl} thiophene-2-carboxamide may be used in an amount of 5 to 20 wt.-%, preferably 8 to 15 wt.-%, more preferably 11 to 12 wt.-%, based on the total amount of the pharmaceutical composition.

The mixture as defined above may further comprise at least one surfactant, wherein the surfactant is selected from the group consisting of anionic, cationic, nonionic and amphoteric surfactants. Typically, the surfactant is selected from the group consisting of sodium salts of fatty alcohol sulfates, particularly sodium lauryl sulfate or sodium dioctylsulfosuccinate; partial fatty acid esters of polyhydric alcohols, particularly glycerol; partial fatty acid esters of sorbitan, particularly sorbitan; partial fatty acid esters of polyhydroxyethy-lensorbitans, particularly polyethylene glycol sorbitan monolaurate, monostearate or monooleate; ethoxylated triglycerides, and any mixtures thereof. In a preferred embodiment the surfactant is sodium lauryl sulfate.

In step a) as defined herein, the surfactant may be used in an amount of 0.2 to 1.0 wt.-%, preferably 0.4 to 0.8 wt.-%, more preferably 0.6 to 0.7 wt.-%, based on the total amount of the pharmaceutical composition. In one preferred embodiment, the binder is a cellulose, preferably hydroxypropyl methyl cellulose, and the surfactant is a salt of a fatty alcohol sulfate, preferably sodium lauryl sulfate.

The mixture as defined above may further comprise at least one filler. The filler may be selected from the group consisting of lactose, dextrose, maltose, sucrose, glucose, fructose, mannitol, maltitol, sorbitol, xylitol, cellulose powder, microcrystalline cellulose, dicalcium phosphate, tricalcium phosphate, magnesium trisilicate, and any mixtures thereof. If the filler is a sugar, it may be present in anhydrous form or as a hydrate, for example as a monohydrate). Typically, the filler is used in an amount of 25 to 40 wt.-%, preferably 30 to 38 wt.-%, more preferably 32 to 34 wt.-%, based on the total amount of the pharmaceutical composition.

Accordingly, the mixture may comprise (S)-5-chloro-N-{[2-oxo-3-[4-(3-oxomorpholin-4-yl)phenyl]oxazolidin-5-yl]methyl} thiophene-2-carboxamide, a hydrophilic binder, a surfactant and optionally a filler. Then, the binder may be a cellulose, particularly hydroxypropyl methyl cellulose, the surfactant may be a salt of a fatty alcohol sulfate, particularly sodium lauryl sulfate, and the filler may be lactose.

Step a) as defined herein may be carried out by dry or by wet milling, wherein dry milling is preferred. Typically, the milling is carried out in a ball mill, a rod mill, an autogenous mill, a SAG mill, high pressure milling rolls, a pebble mill, a Vertical shaft impactor mill, or a tower mil.

In step a) the milling is typically carried out for 10 to 120 min, preferably for 15 to 80 min, more preferably for 30 to 60 min or 40 to 70 min. However, it is also possible that milling times up to some days are used, e.g. when bigger ball mills are used for the production at a commercial scale. Therefore, the milling may also be carried out for 1 to 48 hours, 2 to 24 hours, or 4 to 12 hours. The milling is typically carried out at 20 to 80 rpm, preferably at 40 to 60 rpm, more preferably at 50 rpm. Particularly, the milling may be carried out in a ball mill at 40 to 60 rpm, preferably at 50 rpm, for 40 to 70 min, preferably 60 min.

### Step b)

The process as defined herein may further comprise a step of b) adding at least one excipient to the mixture, preferably directly or indirectly after step a). The excipient or the excipient mixture may be any excipient or any excipient mixture commonly used in the art.

Typically, the at least one excipient added in step b) is selected from the group consisting of a filler, a disintegrant, a lubricant, and any mixture thereof, and is preferably a mixture of a filler, a disintegrant and a lubricant. Therefore, the mixture used in step a) may in some embodiments e.g. already contain a filler, wherein a further filler is added as the excipient or as part of the excipient mixture in step b). When the the at least one excipient added in step b) is a filler or comprises a filler, it may be selected from the group consisting of microcrystalline cellulose, lactose, dextrose, maltose, sucrose, glucose, fructose, mannitol, maltitol, sorbitol, xylitol, cellulose powder, dicalcium phosphate, tricalcium phosphate, magnesium trisilicate, and any mixtures thereof. When the at least one excipient added in step b) is a disintegrant or comprises a disintegrant, it may be selected from the group consisting of croscarmellose, particularly croscarmellose sodium, carboxymethylcellulose, crosslinked polyvinylpyrrolidone, low-substituted hydroxypropylcellulose, partially hydrolysed starch, wheat starch, corn starch, rice starch, potato starch, and any mixture thereof. When the at least one excipient added in step b) is a lubricant or comprises a lubricant, it may be selected from the group consisting of magnesium stearate, fumaric acid, stearic acid, calcium stearate, sodium stearyl fumarate, high molecular weight fatty alcohols, polyethylene glycols, talc, colloidal silica, magnesium oxide, magnesium carbonate, calcium silicate, and any mixture thereof. Particularly, a mixture of croscarmellose sodium, magnesium stearate and microcrystalline cellulose may be added in step b). Thus in one preferred embodiment, the at least one excipient added in step b) is a mixture of croscarmellose sodium, magnesium stearate and microcrystalline cellulose.

When the at least one excipient added in step b) is a disintegrant or comprises a disintegrant, it may be used in an amount of 1 to 10 wt.-%, preferably 2 to 8 wt.-%, more preferably 3 to 4 wt.-%, based on the total amount of the pharmaceutical composition. When the at least one excipient added in step b) is a lubricant or comprises a lubricant, it may be used in an amount of 0.1 to 1.5 wt.-%, preferably 0.5 to 1.0 wt.-%, more preferably 0.7 to 0.8 wt.-%, based on the total amount of the pharmaceutical composition. When the at least one excipient added in step b) is a filler or comprises a filler it may be used in an amount adding up to 100% of the pharmaceutical composition. Typically, it is used in an amount of 40 to 60 wt.-%, preferably 45 to 55 wt.-%, more preferably 47 to 50 wt.-%, based on the total amount of the pharmaceutical composition.

### Step c)

The process as defined herein may further comprise a step of c) sieving the mixture, preferably directly or indirectly after step b). Sieving the mixture in step c) is preferably performed by means of a sieve. Said sieve typically has a mesh size of 0.5 to 2.0 mm, particularly 0.6 to 1.0 mm, more particularly 0.8 mm.

### Step d)

The process as defined herein may further comprise a step of d) mixing the mixture, preferably directly or indirectly after step c). However, step d) may also be carried out directly after step b) as defined above. The mixing may be carried out in a free-fall mixer, a ribbon blender, a continuous processor, a cone screw blender, a screw blender, a double cone blender, a double planetary mixer, a counter-rotating mixer or a paddle mixer. Typically, the mixing is carried out for 5 to 25 min, preferably for 10 to 20 min, more preferably for 15 min. Particularly, step d) may be carried out in a free-fall mixer for 10 to 20 min, more preferably for 15 min. When a free-fall mixer is used, the mixing may be carried out at 3 to 50 rpm, preferably at 10 to 25 rpm, more preferably at 16 rpm.

### Step e)

The process as defined herein may further comprise a step of e) pressing the mixture into tablets, preferably directly or indirectly after step d). However, pressing the mixture into tablets may also be carried out after step a), after step b) or after step c). The pressing into tablets is typically carried out at a pressure of 2 to 40 KN, preferably 2 to 30 KN, and more preferably 3 to 25 KN. After the pressing, the composition is typically orally administrable.

Particularly, the process as defined herein may comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler, and
b) adding at least one excipient; preferably a filler, a disintegrant and a lubricant; to the mixture after step a).

The process as defined herein may also comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler;
b) adding at least one excipient; preferably a filler, a disintegrant and a lubricant; to the mixture after step a); and
c) sieving the mixture after step b).

Further, the process as defined herein may also comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler;
b) adding at least one excipient; preferably a filler, a disintegrant and a lubricant; to the mixture after step a);
c) sieving the mixture after step b);
d) mixing the mixture after step c).

The process as defined herein may also comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler;
e) pressing the mixture into tablets after step a). The pharmaceutical composition produced by such a process is typically orally administrable.

Further, the process as defined herein may also comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler;
b) adding at least one excipient; preferably a filler, a disintegrant and a lubricant; to the mixture after step a);
d) mixing the mixture after step b); and
e) pressing the mixture into tablets after step d). The pharmaceutical composition produced by such a process is typically orally administrable.

Particularly, the process as defined herein may also comprise
a) milling, preferably dry milling, a mixture consisting of (S)-5-chloro-N-{[2-oxo-3-[4-(3-oxomorpholin-4-yl)phenyl]oxazolidin-5-yl]methyl} thiophene-2-carboxamide, a hydrophilic binder, optionally a surfactant and optionally a filler;
b) adding at least one excipient; preferably a filler, a disintegrant and a lubricant; to the mixture after step a);
c) sieving the mixture after step b);
d) mixing the mixture after step c);
e) pressing the mixture into tablets after step d). The pharmaceutical composition produced by such a process is typically orally administrable.

More particularly, the process as defined herein may comprise
a) dry milling a mixture consisting of (S)-5-chloro-N-{[2-oxo-3-[4-(3-oxomorpholin-4-yl)phenyl]oxazolidin-5-yl]methyl} thiophene-2-carboxamide, lactose, sodium laurylsulfate and hydroxypropyl methyl cellulose in a ball mill at 50 rpm for 60 min;
b) adding croscarmellose sodium, microcrystalline cellulose, magnesium stearate to the mixture after step a);
c) sieving the mixture through a sieve with a mesh size of 0.8 mm after step b);
d) mixing the mixture in a free-fall mixer at 16 rpm for 15 min after step c);
e) pressing the mixture into tablets after step d) at a pressure of 5 KN. The pharmaceutical composition produced by such a process is typically orally administrable.

The invention will be more fully understood by references to the following examples. They should not, however, be construed as limiting the scope of the invention. The disclosure of all literature and patent citations mentioned herein is expressly incorporated by reference.

### Examples

### Example 1

2.94 g (S)-5-chloro-N-{[2-oxo-3-[4-(3-oxomorpholin-4-yl)phenyl]oxazolidin-5-yl]methyl} thiophene-2-carboxamide, 8.21 g lactose, 0.15 g sodium laurylsulfate, and 0.88 g hydroxypropyl methyl cellulose are dry milled in ball mill (Retsch S1000; milling container: volume 50ml/Achat body/ + 20 Achatballs as milling tools with a 10mm diameter) at 50 rpm;
0.88 g croscarmellose sodium, 11.76 mg microcrystalline cellulose and 0.18 mg magnesium stearate are added;
the obtained mixture is sieved through a sieve with a mesh size of 0.8 mm;
then mixed in a free-fall mixer at 16 rpm for 15 min;
and then pressed to tablets (6 mm) at a pressure of 5 - 20 kN.

The following conditions were used for the milling step:

| Example: | milling time [min]: |
|---|---|
| 1a | 15 |
| 1b | 30 |
| 1c | 60 |

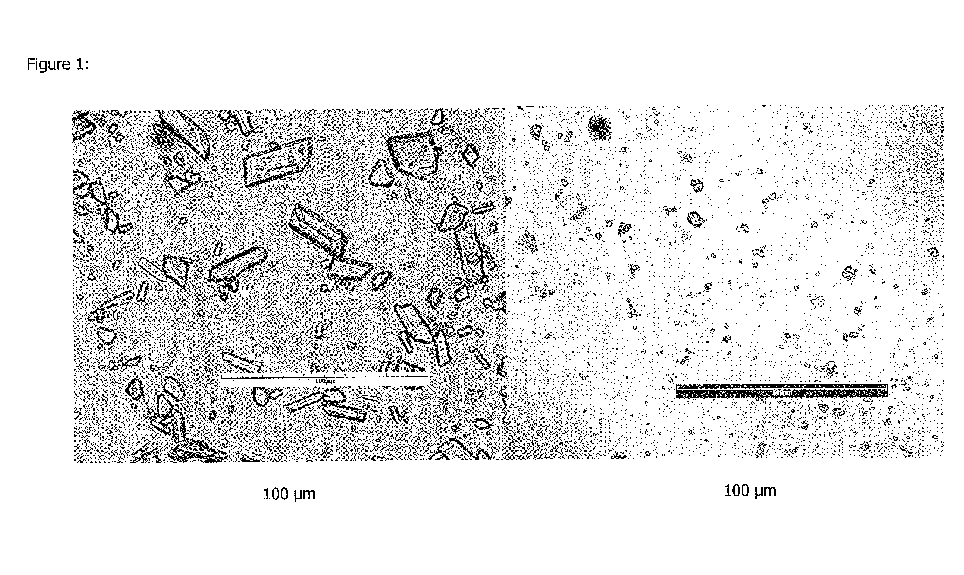

Figure 1 shows light optical microscope images of untreated riviroxaban (left) and a composition comprising (S)-5-chloro-N-{[2-oxo-3-[4-(3-oxomorpholin-4-yl)phenyl]-oxazolidin-5-yl]methyl} thiophene-2-carboxamide, lactose, sodium laurylsulfate, and hydroxypropyl methyl cellulose after milling for 30 min.

### Example 2 (Comparative)

Example 2 was carried out analogous to example 1 with the difference that (S)-5-chloro-N-{[2-oxo-3-[4-(3-oxomorpholin-4-yl)phenyl]oxazolidin-5-yl]methyl} thiophene-2-carboxamide, lactose, sodium laurylsulfate, and hydroxypropyl methyl cellulose were not milled but mixed in a free-fall mixer at 16 rpm for 15 min.

### Example 3 Comparison of the solubility

1 tablet á 85 mg comprising 10.1 mg rivaroxaban prepared according to example 1a,
1 tablet á 85 mg comprising 10.1 mg rivaroxaban prepared according to example 1b,
1 tablet á 85 mg comprising 10.1 mg rivaroxaban prepared according to example 1c, 10 mg rivaroxaban (untreated), and
1 tablet Xarelto^{®} comprising around 10.1 mg rivaroxaban
have been dissolved/suspended in 50 ml acetate puffer solution pH 4.5, at 37°C.
After 2 and 24h the suspension was filtered (pore size 0.45 µm) and the dissolved amount was detected by HPLC.

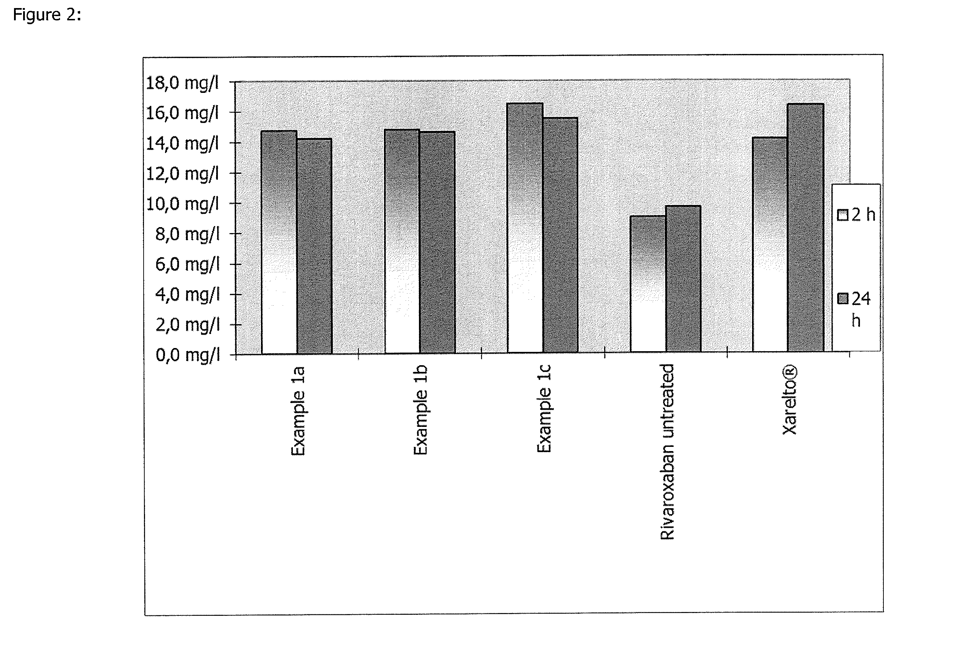

Figure 2 shows a comparison of the solubility. In particular, the compositions according to the invention exhibit solubility's, which are similar to the compositions of the prior art and are improved compared to pure rivaroxaban.

### Example 4, Comparison of the dissolution rate (average value of 3)

3 times 1 tablet comprising 9.6 mg rivaroxaban prepared according to example 1a,
3 times 1 tablet comprising 9.4 mg rivaroxaban prepared according to example 1b,
3 times 1 tablet comprising 9.1 mg rivaroxaban prepared according to example 1c,
3 times 1 tablet comprising 10.6 mg rivaroxaban prepared according to example 2, and 3 times 1 tablet Xarelto^{®} comprising around 10.1 mg rivaroxaban
have been suspended in 900 ml acetate puffer solution, pH 4.5, with a paddle speed of 75 +/- 3 rpm at 37.5 +/- 0.5 °C (dissolution test described in the USP Apparatus 1)

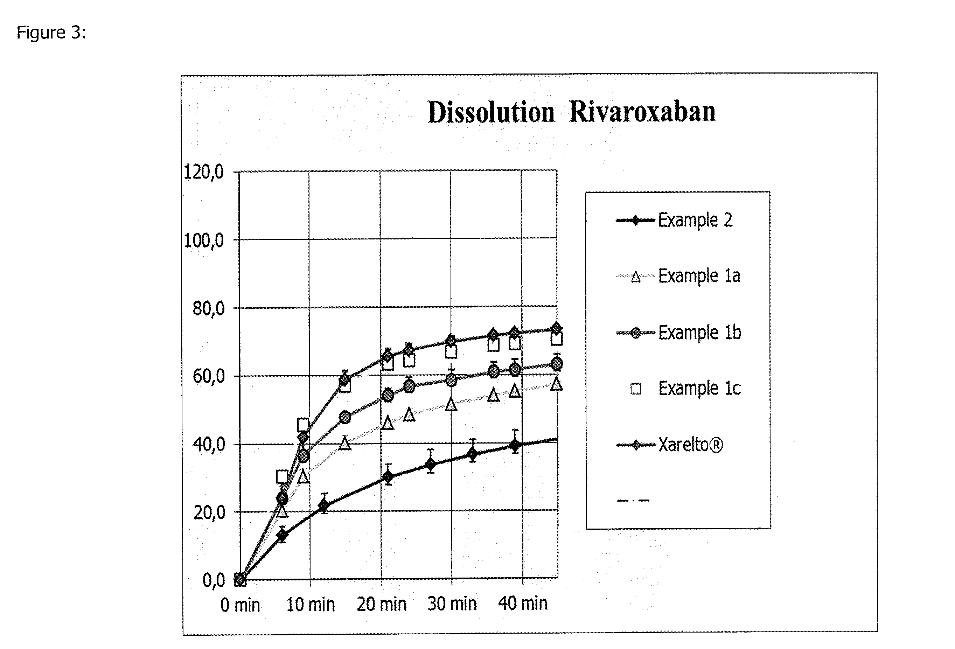

Figure 3 shows a comparison of the dissolution rate. In particular, the compositions according to examples 1a, 1b, and 1c exhibit dissolution rates, which are similar to the compositions of the prior art and are improved compared to the composition according to example 2, in which the ingredients have only been mixed.

### Example 5, Comparison of the solubility

1 tablet comprising 9.1 mg rivaroxaban prepared according to example 1c,
1 tablet comprising 10.6 mg rivaroxaban prepared according to example 2,
1 tablet Xarelto^{®} comprising around 10.1 mg rivaroxaban, and
1 tablet prepared by spray drying a mixture of sodium laurylsulfate, hydroxypropyl methyl cellulose, (S)-5-chloro-N-{[2-oxo-3-[4-(3-oxomorpholin-4-yl)phenyl]oxazolidin-5-yl]methyl} thiophene-2-carboxamide and water on lactose, mixed with croscarmellose sodium, microcrystalline cellulose and magnesium stearate, and then pressed to tablets comprising 10.1 mg rivaroxaban;
have been dissolved/suspended in 50 ml acetate puffer solution pH 4.5, at 37°C.
After 2 and 24h the suspension was filtered (pore size 0.45 µm) and the dissolved amount was detected by HPLC.

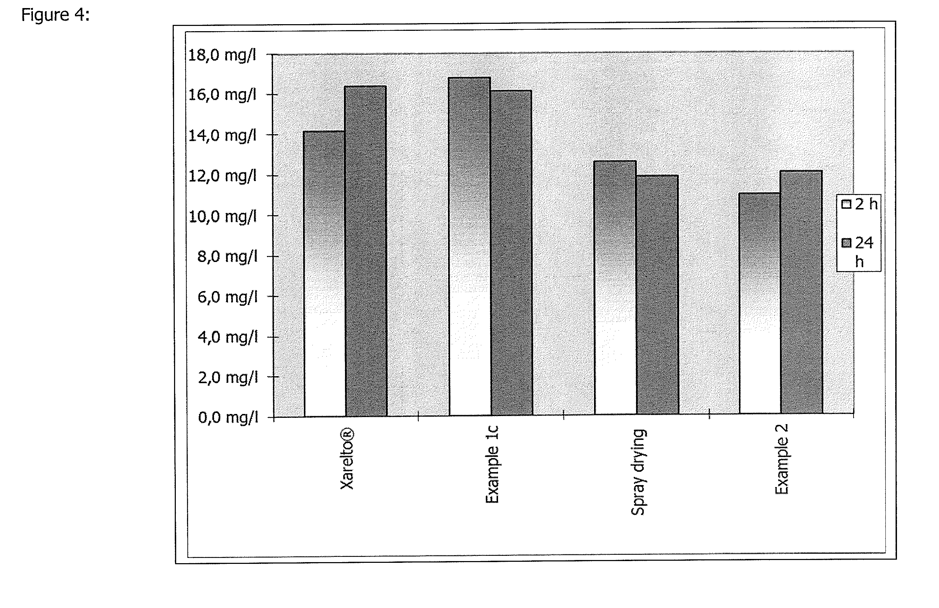

Figure 4 shows a comparison of the solubility. In particular, the composition according to the invention exhibits a solubility, which is similar to the compositions of the prior art and is improved compared to a composition, in which the ingredients have only been mixed or spray dried.

### Example 6, Comparison of the dissolution rate (average value of 3)

3 times 1 tablet comprising 9.1 mg rivaroxaban prepared according to example 1c,
3 times 1 tablet comprising 10.6 mg rivaroxaban prepared according to example 2,
3 times 1 tablet Xarelto^{®} comprising around 10.1 mg rivaroxaban, and
3 times 1 tablet prepared by spray drying a mixture of sodium laurylsulfate, hydroxypropyl methyl cellulose, (S)-5-chloro-N-{[2-oxo-3-[4-(3-oxomorpholin-4-yl)phenyl]oxazolidin-5-yl]methyl} thiophene-2-carboxamide and water on lactose, mixed with croscarmellose sodium, microcrystalline cellulose and magnesium stearate, and then pressed to tablets comprising 10.1 mg rivaroxaban;
have been suspended in 900 ml acetate puffer solution, pH 4.5, with a paddle speed of 75 +/- 3 rpm at 37.5 +/- 0.5 °C (dissolution test described in the USP Apparatus 1)

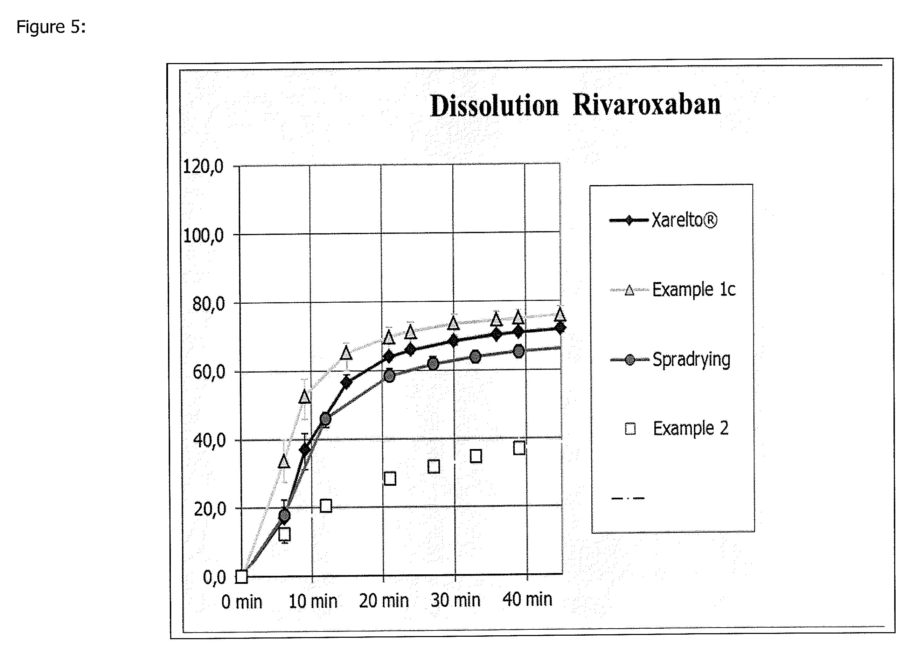

Figure 5 shows a comparison of the dissolution rate. In particular, the composition according to example 1c exhibit a dissolution rate, which is similar to the compositions of the prior art and is improved compared to compositions according to example 2, in which the ingredients have only been mixed or spray dried.

The following pages of the description refer to the embodiments of the invention listed as separate items:
1. A process for the preparation of a pharmaceutical composition comprising (S)-5-chloro-N-{[2-oxo-3-[4-(3-oxomorpholin-4-yl)phenyl]oxazolidin-5-yl]methyl} thiophene-2-carboxamide, wherein the process comprises a step of
   a) milling a mixture comprising (S)-5-chloro-N-{[2-oxo-3-[4-(3-oxomorpholin-4-yl)phenyl]oxazolidin-5-yl]methyl} thiophene-2-carboxamide and at least one hydrophilic binder.
2. The process of item 1, wherein the binder is water soluble.
3. The process of item 1 or 2, wherein the binder is selected from the group consisting of hydroxypropyl methyl cellulose, cellulose methyl ether, hydroxypropyl cellulose, ethyl cellulose, carboxymethyl cellulose, galactomannan gum, xanthan, glycerides, acrylic and methacrylic copolymers with trimethylammoniomethyl acrylate, copolymers of dimethylaminomethacrylic acid and neutral methacrylic acid esters, and polymers of methacrylic acid or methacrylic acid, and any mixtures thereof, and is preferably hydroxypropyl methyl cellulose.
4. The process of any of items 1 to 3, wherein the binder is used in an amount of 2 to 10 wt.-%, preferably 3 to 8 wt.-%, more preferably 3 to 4 wt.-%, based on the total amount of the pharmaceutical composition.
5. The process of any of items 1 to 4, wherein (S)-5-chloro-N-{[2-oxo-3-[4-(3-oxomorpholin-4-yl)phenyl]oxazolidin-5-yl]methyl) thiophene-2-carboxamide is used in an amount of 5 to 20 wt.-%, preferably 8 to 15 wt.-%, more preferably 11 to 12 wt.-%, based on the total amount of the pharmaceutical composition.
6. The process of any of items 1 to 5, wherein the mixture further comprises at least one surfactant.
7. The process of item 6, wherein the surfactant is selected from the group consisting of anionic, cationic, nonionic and amphoteric surfactants.
8. The process of item 6 or 7, wherein the surfactant is selected from the group consisting of sodium salts of fatty alcohol sulfates, particularly sodium lauryl sulfate or sodium dioctylsulfosuccinate; partial fatty acid esters of polyhydric alcohols, particularly glycerol; partial fatty acid esters of sorbitan, particularly sorbitan; partial fatty acid esters of polyhydroxyethy-lensorbitans, particularly polyethylene glycol sorbitan monolaurate, monostearate or monooleate; ethoxylated triglycerides, and any mixtures thereof, and is preferably sodium lauryl sulfate.
9. The process of any of items 6 to 8, wherein the surfactant is used in an amount of 0.2 to 1.0 wt.-%, preferably 0.4 to 0.8 wt.-%, more preferably 0.6 to 0.7 wt.-%, based on the total amount of the pharmaceutical composition.
10. The process of any of items 1 to 9, wherein the mixture further comprises at least one filler.
11. The process of item 10, wherein the at least one filler is selected from the group consisting of lactose, dextrose, maltose, sucrose, glucose, fructose, mannitol, maltitol, sorbitol, xylitol, cellulose powder, microcrystalline cellulose, dicalcium phosphate, tricalcium phosphate, magnesium trisilicate, and any mixtures thereof, and is preferably lactose.
12. The process of item 10 or 11, wherein the at least one filler is used in an amount of 25 to 40 wt.-%, preferably 30 to 38 wt.-%, more preferably 32 to 34 wt.-%, based on the total amount of the pharmaceutical composition.
13. The process of any of items 1 to 12, wherein step a) is carried out by dry or by wet milling, preferably dry milling.
14. The process of any of items 1 to 13, wherein in step a) the milling is carried out in a ball mill, a rod mill, an autogenous mill, a SAG mill, high pressure milling rolls, a pebble mill, a Vertical shaft impactor mill, or a tower mil.
15. The process of any of items 1 to 14, wherein in step a) the milling is carried out for 10 to 120 min, preferably for 15 to 80 min, more preferably for 30 to 60 min.
16. The process of any of items 1 to 15, wherein in step a) the milling is carried out at 20 to 80 rpm, preferably at 40 to 60 rpm, more preferably at 50 rpm.
17. The process of any of items 1 to 16, wherein the process further comprises a step of b) adding at least one excipient to the mixture, preferably after step a).
18. The process of item 17, wherein the at least one excipient is selected from the group consisting of a filler, a disintegrant, a lubricant, and any mixture thereof, and is preferably a mixture of a filler, a disintegrant and a lubricant.
19. The process of item 18, wherein the filler is selected from the group consisting of microcrystalline cellulose, lactose, dextrose, maltose, sucrose, glucose, fructose, mannitol, maltitol, sorbitol, xylitol, cellulose powder, microcrystalline cellulose, dicalcium phosphate, tricalcium phosphate, magnesium trisilicate, and any mixtures thereof.
20. The process of item 18 or 19, wherein the disintegrant is selected from the group consisting of croscarmellose, particularly croscarmellose sodium, carboxymethylcellulose, crosslinked polyvinylpyrrolidone, low-substituted hydroxypropylcellulose, partially hydrolysed starch, wheat starch, corn starch, rice starch, potato starch, and any mixture thereof.
21. The process of any of items 18 to 20, wherein the disintegrant is used in an amount of 1 to 10 wt.-%, preferably 2 to 8 wt.-%, more preferably 3 to 4 wt.-%, based on the total amount of the pharmaceutical composition.
22. The process of any of items 18 to 21, wherein the lubricant is selected from the group consisting of magnesium stearate, fumaric acid, stearic acid, calcium stearate, sodium stearyl fumarate, high molecular weight fatty alcohols, polyethylene glycols, talc, colloidal silica, magnesium oxide, magnesium carbonate, calcium silicate, and any mixture thereof.
23. The process of any of items 18 to 22, wherein the lubricant is used in an amount of 0.1 to 1.5 wt.-%, preferably 0.5 to 1.0 wt.-%, more preferably 0.7 to 0.8 wt.-%, based on the total amount of the pharmaceutical composition.
24. The process of any of items 1 to 23, wherein the process further comprises a step of c) sieving the mixture, preferably after step b).
25. The process of item 24, wherein the sieving in step c) is carried out with a sieve having a mesh size of 0.5 to 2.0 mm, particularly 0.6 to 1.0 mm, more particularly 0.8 mm.
26. The process of any of items 1 to 25, wherein the process further comprises a step of d) mixing the mixture, preferably after step c).
27. The process of item 26, wherein in step d) the mixing is carried out in a free-fall mixer, a ribbon blender, a continuous processor, a cone screw blender, a screw blender, a double cone blender, a double planetary mixer, a counter-rotating mixer or a paddle mixer.
28. The process of item 26 or 27, wherein in step d) the mixing is carried out for 5 to 25 min, preferably for 10 to 20 min, more preferably for 15 min.
29. The process of any of items 1 to 28, wherein the process further comprises a step of e) pressing the mixture into tablets, preferably after step d).
30. The process of item 29, wherein the pressing into tablets is carried out at a pressure of 2 to 40 KN, preferably 2 to 30, and more preferably 3 to 25 KN.
31. The process of any of items 1 to 30, wherein the process comprises
   a) dry milling a mixture consisting of (S)-5-chloro-N-{[2-oxo-3-[4-(3-oxomorpholin-4-yl)phenyl]oxazolidin-5-yl]methyl} thiophene-2-carboxamide, lactose, sodium laurylsulfate, hydroxypropyl methyl cellulose in a ball mill at 50 rpm for 60 min;
   b) adding croscarmellose sodium, microcrystalline cellulose, magnesium stearate to the mixture after step a);
   c) sieving the mixture through a sieve with a mesh size of 0.8 mm after step b);
   d) mixing the mixture in a free-fall mixer at 16 rpm for 15 min after step c);
   e) pressing the mixture into tablets after step d) at a pressure of 5 KN.
32. The process of any of items 1 to 31, wherein the process does not include granulating (S)-5-chloro-N-{[2-oxo-3-[4-(3-oxomorpholin-4-yl)phenyl]oxazolidin-5-yl]methyl} thiophene-2-carboxamide or granulating a mixture comprising (S)-5-chloro-N-{[2-oxo-3-[4-(3-oxomorpholin-4-yl)phenyl]oxazolidin-5-yl]methyl} thiophene-2-carboxamide.
33. The process of any of items 29 to 32, wherein the pharmaceutical composition is orally administrable.

### List of references

WO2005/060940
Lerk, Lagash, Fell, Nauta, Journal of Pharmaceutical Sciences, Vol 67, no. 7, July 1978, 935-939: "Effect of Hydrophilization of hydrophobicity Drugs on release rate from Capsules".
Lerk, Lagash, Lie-A-Huen, Broersma, Zuurman, Journal of Pharmaceutical Sciences, Vol 68, no. 5, May 1979, 634-638: "In Vitro and In Vivo Availability of Hydrophilized from phenytoin capsules".

## Claims

1. A process for the preparation of a pharmaceutical composition comprising (S)-5-chloro-N-{[2-oxo-3-[4-(3-oxomorpholin-4-yl)phenyl]oxazolidin-5-yl]methyl} thiophene-2-carboxamide, wherein the process comprises a step of
a) milling a mixture comprising (S)-5-chloro-N-{[2-oxo-3-[4-(3-oxomorpholin-4-yl)phenyl]oxazolidin-5-yl]methyl} thiophene-2-carboxamide and at least one hydrophilic binder.

2. The process of claim 1, wherein the binder is water soluble.

3. The process of claim 1 or 2, wherein the binder is selected from the group consisting of hydroxypropyl methyl cellulose, cellulose methyl ether, hydroxypropyl cellulose, ethyl cellulose, carboxymethyl cellulose, galactomannan gum, xanthan, glycerides, acrylic and methacrylic copolymers with trimethylammoniomethyl acrylate, copolymers of dimethylaminomethacrylic acid and neutral methacrylic acid esters, polymers of methacrylic acid or methacrylic acid, and any mixtures thereof.

4. The process of any of claims 1 to 3, wherein the mixture further comprises at least one surfactant.

5. The process of claim 4, wherein the surfactant is selected from the group consisting of sodium salts of fatty alcohol sulfates, partial fatty acid esters of polyhydric alcohols, partial fatty acid esters of sorbitan, partial fatty acid esters of polyhydroxyethy-lensorbitans, ethoxylated triglycerides, and any mixtures thereof.

6. The process of any of claims 1 to 5, wherein the mixture further comprises at least one filler.

7. The process of any of claims 1 to 6, wherein step a) is carried out by dry or by wet milling.

8. The process of any of claims 1 to 7, wherein the process further comprises a step of b) adding at least one excipient to the mixture, preferably after step a).

9. The process of claim 8, wherein the at least one excipient is selected from the group consisting of a filler, a disintegrant, a lubricant, and any mixture thereof.

10. The process of any of claims 1 to 9, wherein the process further comprises a step of c) sieving the mixture, preferably after step b).

11. The process of claim 10, wherein the sieving in step c) is carried out with a sieve having a mesh size of 0.5 to 2.0 mm, particularly 0.6 to 1.0 mm, more particularly 0.8 mm.

12. The process of any of claims 1 to 11, wherein the process further comprises a step of d) mixing the mixture, preferably after step c).

13. The process of any of claims 1 to 12, wherein the process further comprises a step of e) pressing the mixture into tablets, preferably after step d).

14. The process of any of claims 1 to 13, wherein the process does not include granulating (S)-5-chloro-N-{[2-oxo-3-[4-(3-oxomorpholin-4-yl)phenyl]oxazolidin-5-yl]methyl} thiophene-2-carboxamide or granulating a mixture comprising (S)-5-chloro-N-{[2-oxo-3-[4-(3-oxomorpholin-4-yl)phenyl]oxazolidin-5-yl]methyl} thiophene-2-carboxamide.

15. The process of claim 13 or 14, wherein the pharmaceutical composition is orally administrable.
